# EUROPÄISCHE PATENTSCHRIFT

(11) **EP 1 200 561 B1**
(45) Veröffentlichungstag und Bekanntmachung des Hinweises auf die Patenterteilung: **14.06.2006**
(21) Anmeldenummer: 99939251.7
(22) Anmeldetag: 05.08.1999
(51) Int. Cl.: C12N 5/10, C12P 21/02, C12N 9/64, C07K 14/745

(54) **REKOMBINANTER STABILER ZELLKLON, SEINE HERSTELLUNG UND VERWENDUNG**
RECOMBINANT STABILE CELL CLONE, ITS PRODUCTION AND USE THEREOF
CLONE CELLULAIRE RECOMBINE STABLE, SA PRODUCTION ET SON UTILISATION

(43) Veröffentlichungstag der Anmeldung: 02.05.2002
(73) Patentinhaber: Baxter Aktiengesellschaft, 1221 Wien (AT)
(72) Erfinder: REITER, Manfred, A-1150 Wien (AT); MUNDT, Wolfgang, A-1080 Vienna (AT); DORNER, Friedrich, A-1230 Wien (AT)
(74) Vertreter: Perrey, Ralf
(86) Internationale Anmeldenummer: PCT/AT1999/000197
(87) Internationale Veröffentlichungsnummer: WO 2001/011021

(56) Entgegenhaltungen:
- EP-A- 0 666 312
- WO-A-96/15231
- WO-A-96/18734
- WO-A-97/05240
- US-A- 5 633 162
- US-A- 5 851 800
- ZANG M ET AL: "PRODUCTION OF RECOMBINANT PROTEINS IN CHINESE HAMSTER OVARY CELLS USING A PROTEIN-FREE CELL CULTURE MEDIUM" BIO/TECHNOLOGY,US,NATURE PUBLISHING CO. NEW YORK, Bd. 13, 1. April 1995 (1995-04-01), Seiten 389-392, XP002032677 ISSN: 0733-222X
- HARANT H ET AL: "Two-dimensional electrophoresis as a tool for control of quality and consistency in production systems using animal cells" CYTOTECHNOLOGY, Bd. 6, 1992, Seiten 119-127, XP002140916
- KATINGER, H. ET AL: "Long-term stability of continuously perfused animal cells immobilized on novel macroporous microcarriers" ADV. MOL. CELL BIOL. (1996), 15A(BIOCHEMICAL TECHNOLOGY), 193-207 , XP000914905
- FISCHER, BERNHARD ET AL: "Comparison of N-glycan pattern of recombinant human coagulation factors II and IX expressed in chinese hamster ovary (CHO) and African green monkey (Vero) cells" J. THROMB. THROMBOLYSIS (1996), 3(1), 57-62 , XP000914907
- FISCHER B ET AL: "Structural analysis of recombinant von Willebrand factor: identification of hetero- and homo-dimers" FEBS LETTERS, Bd. 351, 1994, Seiten 345-348, XP002140917 in der Anmeldung erwähnt
- KAUFMAN RJ ET AL: "Effect of von Willebrand Factor coexpression on the synthesis and secretion of Factor VIII in Chinese Hamster Ovary Cells" MOLECULAR AND CELLULAR BIOLOGY, Bd. 9, Nr. 3, 1989, Seiten 1233-1242, XP002140918

## Beschreibung

Die vorliegende Erfindung betrifft einen stabilen rekombinanten Zellklon, der im serum- und protein-freien Medium in Abwesenheit eines Selektionsdruckes für mindestens 40 Generationen stabil ist, eine Biomasse erhalten durch Vermehrung des stabilen Zellklons unter serum- und proteinfreien Kultivierungsbedingungen und ein Verfahren zur Herstellung von rekombinanten Proteinen mittels der Biomasse. Desweiteren betrifft die Erfindung ein Verfahren zur Gewinnung von stabilen rekombinanten Zellklonen. Weiters betrifft die Erfindung die Herstellung eines rekombinanten Proteins in einem serum- und protein- freien synthetischen Minimalmedium.

Die Herstellung von rekombinanten Proteinen, insbesondere von biomedizinischen Produkten, wie Blutfaktoren, gewinnt immer mehr an Bedeutung. Um ein optimales Wachstum von rekombinanten Zellen zu ermöglichen, wird dem Medium zumeist Serum zugegeben. Aufgrund der hohen Kosten von Serum und um mögliche Verunreinigungen durch virale oder molekulare Pathogene durch das Serum im Kultivierungsmedium zu vermeiden, wurde eine Reihe von serumfreien Medien entwickelt, die insbesondere keine Zusätze bovinen oder humanen Ursprungs enthalten sollten. Die Verwendung solcher Medien beim Herstellungsprozeß erlaubt neben der geringen Kontaminationsgefahr der hergestellten Produkte durch virale und molekulare Pathogene auch eine einfachere Reinigung der exprimierten Proteine.

Rekombinante Zellen werden zumeist in serumhaltigem Medium bis zu einer hohen Zelldichte, etwa für eine "Working cell bank" angezogen, und anschließend während der Produktionsphase auf serumfreies Medium umadaptiert.

Miyaji et al. (1990, Cytotechnology 3:133-140) selektionierten serum-unabhängige Zellklone in serumfreiem Medium, das Insulin und Transferrin enthielz. Es zeigte sich jedoch, daß nach 15 Tagen die Lebendzellzahl und die Expressionsrate ständig abnahm. Durch Coamplifikation mit einem Markergen versuchten Miyaji et al. (1990, Cytotechnology 4:173-180) die Expressionsrate und die Produktivität der rekombinanten Zellen zu verbessern.

Yamauchi et al. (1992, Biosci. Biotechnol. Biochem. 56:600-604) etablierten serum-unabhängige rekombinante CHO-Subklone durch Kultivierung serum-abhängiger Zellen auf Mikrotiterplatten als Monolayer für 3 bis 4 Wochen in serum-freiem Medium, das humanes Serumalbumin, Insulin und Transferrin enthielt. Etwa 0,1% der Zellen waren serum-unabhängig. Ein Teil der Subklone wuchs auch in Suspensionkultur in serumfreiem Medium, wobei die Zellen jedoch aggregierten und klumpten. Die Verdopplungszeit der Zellen betrug 1,5 Tage. Es werden jedoch weder Angaben über die Stabilität der erhaltenen serum-unabhängigen Klone noch über die Langzeitkultivierung dieser Klone unter serumfreien Bedingungen gemacht.

Medien, die die Aufrechterhaltung der metabolischen Aktivität und ein Wachstum von Zellen während der serumfreien Phase erlauben, enthalten oftmals zusätzlich Substanzen, wie Wachstumsfaktoren, wie Insulin oder Transferrin, oder Adhärenzfaktoren, die die Serumkomponenten ersetzen.

Um die Zugabe von Polypeptidfaktoren, wie Insulin oder Transferrin, zu vermeiden und proteinfreie Kultivierungsbedingungen zu erlauben, wurden verschiedene Techniken entwickelt. So wurden speziell definierte, komplette proteinfreie Medien entwickelt, die ein Zellwachstum auch unter proteinfreien Bedingungen erlauben.

WO 97/05240 beschreibt die Herstellung von rekombinanten Proteinen unter proteinfreien Bedingungen, wobei die Zellen neben dem gewünschten Protein einen Wachstumsfaktor coexprimieren.

JP 2696001 beschreibt die Verwendung von proteinfreiem Medium für die Herstellung von Faktor VIII in CHO-Zellen unter Zugabe eines nicht-ionischen Oberflächenmittels oder Cyclodextrin zur Steigerung der Produktivität der Wirtszellen. Um die Wirksamkeit dieser Zusätze zu steigern, wird die Zugabe von beispielsweise Butyrat und Lithium empfohlen.

WO 96/26266 beschreibt die Kultivierung von Zellen in einem Medium, das ein Glutamin enthaltendes Proteinhydrolysat enthält, dessen Gehalt an freien Aminosäuren kleiner als 15% des Gesamtproteingewichts ist und dessen Peptide ein Molekulargewicht von kleiner 44kD aufweisen. Als Kultivierungsmedium für die Zellkulturen wird als Basismedium synthetisches Minimalmedium verwendet, dem neben Proteinhydrolysat auch unter anderem fötales Kälberserum, Gentamycin und Mercaptoethanol zugesetzt wird. Die Verwendung dieses serumhältigen Mediums für die rekombinante Herstellung von Blutfaktoren wird nicht erwähnt.

US 5,393,668 beschreibt spezielle synthetische Oberflächen, die ein Wachstum von adhärenten Zellen unter proteinfreien Bedingungen erlauben.

Um die Zellproliferation zu stimulieren, wurden CHO-Zellen, die humanes Insulin überexprimieren, auf einem artifiziellen Substrat, an das kovalent Insulin gebunden ist, vermehrt (Ito et al., 1996, PNAS USA 93:3598-3601).

Reiter et al. (1992, Cytotechnology 9:247-253) beschreiben die Immobilisierung von in serumhaltigem Medium angezüchteten r-CHO-Zellen bei einer hohen Dichte auf Trägern und anschließender Perfusion der immobilisierten Zellen in proteinfreiem Medium während der Produktionsphase, wobei eine kontinuierliche Freisetzung von Protein in den Zellkulturüberstand festgestellt wurde. Die Zellen wurden dabei jedoch für weniger als 10 Generationen in proteinfreiem Medium perfundiert.

Bisherige Verfahren zur erfolgreichen Herstellung einer großtechnischen "large-scale"-Zellkultur unter proteinfreien Bedingungen werden für kontinuierliche Zellinien, insbesondere VERO-Zellen, beschrieben (WO 96/15231). Die Zellen werden dabei unter serum- und proteinfreien Bedingungen von der Original-Ampulle bis zum großtechnischen Maßstab von 1200 l angezogen. Es handelt sich dabei jedoch nicht um rekombinante Zellen, sondern um Wirtszellen, die für die Produktion von Virusantigen in einem lytischen Prozeß eingesetzt werden.

Im Gegensatz zu adhärenten VERO-Zellen sind beispielsweise CHO-Zellen nur bedingt haftungsabhängig. Mittels konventioneller Methoden unter serumhaltigen Bedingungen angezogene CHO-Zellen können sowohl an glatte als auch an poröse Mikroträger binden (US 4,978,616, Reiter et al., 1992, Cytotechnology 9:247-253) Werden CHO-Zellen unter serumfreien Bedingungen angezogen, so verlieren sie diese Eigenschaft und haften nicht an glatten Trägern, wie etwa Cytodex 3, oder lösen sich leicht von diesen ab, sofern nicht adhärenzfördernde Zusätze, wie beispielsweise Fibronektin, Insulin oder Transferrin ins Medium gegeben werden. Aufgrund der geringen Adhärenz von CHO-Zellen an Träger unter serumfreien Bedingungen erfolgt die Produktion von rekombinanten Proteinen daher zumeist in Suspensionskultur. Der Produktionsprozess kann dabei in einem kontinuierlichen oder batch-weisen Verfahren ablaufen. Die rekombinante Zellkultur wird dabei bis zu einer optimalen Zelldichte im Bioreaktor angezogen, gegebenenfalls die Proteinexpression induziert und zum Ernten das Medium enthaltend die exprimierten Proteine, jedoch auch rekombinante Zellen in gewissen Abständen dem Reaktionstank und damit dem Produktionsprozeß entzogen. Durch den ständigen Verlust an Biomasse sinkt die Produktionseffizienz im Bioreaktor ab und erhöht sich erst nach Zugabe von frischem Medium langsam wieder, da die Zellen auf die gewünschte Zelldichte hochwachsen müssen Es gibt daher, trotz des kontinuierlichen Prozesses, immer wieder eine Verzögerungsphase, in der die Produktionsrate bei diesem System absinkt. Zudem ist die Wachstums- und Produktionskapazität durch die maximal erreichbare Zelldichte in einem solchen System begrenzt.

Bei der Adaptierung von unter serumhaltigen Bedingungen angezüchteten Zellen auf proteinfreies Medium wurde immer wieder festgestellt, daß die Ausbeute an exprimiertem Protein und die Produktivität von rekombinanten CHO-Zellen nach Adaption in proteinfreiem Medium im Vergleich zu serumhaltigen Bedingungen stark absinkt (Paterson et al., 1994, Appl. Microbiol. Biotechnol. 40:691-658).

Dies ist auf eine Instabilität oder reduziertes Wachstum der rekombinanten Klone aufgrund der geänderten Kulturbedingungen zurückzuführen. Aufgrund der veränderten Fermentationsbedingungen wird - trotz Einsetzen eines stabilen Ursprungsklons - immer wieder ein Großteil der Zellen zu Zellen mit verringerter Expression oder auch Nicht-Produzenten, die während des Produktionsprozesses Produkt-Produzenten überwuchern, wodurch die Fermenterkultur letztlich zu einem Großteil aus Nicht-Produzenten bzw. solchen Zellen mit geringer Expression besteht.

Dies hat zur Folge, daß die maximale Produktionskapazität der Fermentationskultur kontinuierlich absinkt und eine maximale Produktproduktion auf eine bestimmte Anzahl von Generationen oder Zellpassagen beschränkt ist.

Im Stand der Technik sind verschiedene Methoden beschrieben, die sich mit der Züchtung von Zellen in Serumfreien oder serum-und proteinfreiem Medium bei Anwesenheit eines Selektions druckes befassen (Zang et al., Biotechnology, Bd. 13, 1. April 1995, Seiten 389-392 ; WO 96 18734 A; Fischer et al., Thromb. Thrombolysis, 1996, 3(1), 57-62; Fischer et al., FEBS Letters, Bd. 351, 1994, Seiten 345-348, Kanfuran et al., Molecular and Cellular Biology, Bol.9, Nr. 3, 1989, Seiten 1233-1242).

Es besteht daher ein Bedarf an einem System, bei dem eine kontinuierliche Produktion, insbesondere bei der großtechnischen Herstellung von rekombinanten Proteinen unter serum- und protein- freien Bedingungen über einen möglichst langen Zeitraum möglich ist.

Es wäre weiterhin wünschenswert, einen rekombinanten Zellklon zu erhalten, der für viele Generationen in der Produktionsphase unter proteinfreien Bedingungen stabil ist und rekombinantes Protein exprimiert.

Aufgabe der vorliegenden Erfindung ist es daher, ein effizientes Verfahren für die Herstellung von rekombinanten Proteinen unter serum-und proteinfreien Kultivierungs- und Produktionsbedingungen zur Verfügung zu stellen.

Eine weiteres Ziel ist es, einen stabilen rekombinanten Zellklon zur Verfügung zu stellen.

Die Aufgabe wird erfindungsgemäß durch die in den beigefügten Ansprüchen gekennzeichneten Ausfünungsformen gelöst.

Der erfindungsgemäße Zellklon bildet daher eine Population von Zellen, die fürmindestens 40 Generationen stabil im serum- und proteinfreien Medium in Abwesenheit eines Selektiondruckes kultiviert werden kann. Vorzugsweise sind dabei mehr als 80%, insbesondere mehr als 99% der Zellen der erfindungsgemäßen Zellpopulation bzw. des erfindungsgemäßen Zellklons für mindestens 40 Generationen stabil.

Die Kultivierung der Zellen erfolgt dabei Ohne Selection, Z.B. ohne Selektion auf das Selektionsmarker- und/oder Amplifikationsgen, beispielsweise in Abwesenheit von MTX bei CHO-dhfr⁻-Zellen.

Unter Ursprungszellklon wird im Rahmen dieser Erfindung ein rekombinanter Zellklon-Transfektant verstanden, der nach Transfektion von Wirtszellen mit einer rekombinanten Nukleotidsequenz unter Laborbedingungen stabil rekombinantes Produkt exprimiert. Der Ursprungsklon wird zur Optimierung des Wachstums in serumhaltigem Medium angezüchtet. Zur Erhöhung der Produktivität wird der Ursprungsklon gegebenenfalls in Gegenwart eines Selektionsmittels und Selektion auf den Selektionsmarker und/oder Amplifikationsmarker angezogen. Für die großtechnische Produktion wird der Ursprungszellklon unter serumhaltigen Kultivierungsbedingungen bis zu einer hohen Zelldichte angezogen und kurz vor der Produktionsphase auf serum- und/oder proteinfreies Medium umadaptiert. Die Kultivierung erfolgt dabei ohne. Selektionsdruck.

Es wurde gefunden, daß unter diesen Bedingungen ein Großteil von mehr als 95% der Zellen in einer solchen auf serum- und proteinfreiem Medium umadaptierten Zellkultur zu Nicht-Produkt-Produzenten werden. Mittels Immunfluoreszenz mit produkt-spezifischen Antikörpern konnte gezeigt werden, daß abhängig von der Generationszeit der Zellen in serum- und proteinfreiem Medium die Anzahl der Nicht-Produzenten in einer Kultur ansteigt und die Produkt-Produzenten überwächst, wodurch die Produktionskapazität der Kultur absinkt.

Die nach Umadaptierung auf serum- und proteinfreies Medium erhaltene Zellkultur wird auf diejenigen Zellklone der Zellpopulation getestet, die unter serum- und proteinfreien Bedingungen, in Abwesenheit eines Selektionsdruckes, stabile Produkte produzieren. Dies kann beispielsweise durch Immunfluoreszenz mit markierten, spezifischen, gegen das rekombinante Polypeptid oder Protein gerichteten Antikörpern erfolgen. Die als Produkt-Produzenten identifizierten Zellen werden aus der Zellkultur isoliert, und unter serum- und proteinfreien, vorzugsweise unter produktionsäquivalenten Bedingungen erneut vermehrt. Die Isolierung der Zellen kann dabei durch Vereinzelung der Zellen und Testen auf Produkt-Produzenten erfolgen. Gegebenenfalls wird die Zellkultur, enthaltend die stabilen Zellen, erneut auf stabile rekombinante Klone getestet und diese aus der Zellkultur isoliert und auskloniert. Anschließend werden die unter serum- und proteinfreien Bedingungen erhaltenen stabilen rekombinanten Zellklone unter serum- und proteinfreien Bedingungen weitervermehrt.

Der erfindungsgemäße rekombinante Zellklon zeichnet sich insbesondere dadurch aus, daß er in serumfreiem und proteinfreiem Medium in Abwesenheit eines Selectiondruckes für mindestens 40, vorzugsweise mindestens 50, insbesondere mehr als 60, Generationen stabil ist und rekombinantes Produkt exprimiert. Dabei zeigen sie diese Stabilität ohne daß sie durch Hilfsmittel wie Matricen oder feste Oberflächen z.B. als Träger unterstützt werden müßten. Auch ist eine Züchtung in hohen Zelldichten erfindungsgemäß nicht erforderlich.

Gemäß einem besonderen Aspekt der Erfindung liegt der stabile rekombinante Zellklon in isolierter Form vor. Ausgehend vom stabilen Zellklon wird unter serum- und proteinfreien Bedingungen eine Zellkultur durch Vermehrung der stabilen Zellen gewonnen.

Der erfindungsgemäße stabile rekombinante Zellklon ist vorzugsweise von einer rekombinanten Säugerzelle abgeleitet. Rekombinante Säugerzellen können dabei alle Zellen sein, die für ein rekombinantes Polypeptid oder Protein kodierende Sequenzen enthalten. Umfaßt sind dabei alle kontinuierlich wachsenden Zellen, die sowohl adhärent als auch nicht-adhärent wachsen. Besonders bevorzugt sind rekombinante CHO-Zellen oder BHK-Zellen. Rekombinante Polypeptide oder Proteine können Blutfaktoren, Wachstumsfaktoren oder andere biomedizinisch relevante Produkte sein.

Gemäß der vorliegenden Erfindung sind stabile rekombinante Zellklone bevorzugt, die die kodierende Sequenz für einen rekombinanten Blutfaktor wie Faktor II, Faktor V, Faktor VII, Faktor VIII, Faktor IX, Faktor X, Faktor XI, Protein S, Protein C, eine aktivierte Form eines dieser Faktoren, oder vWF enthalten, und in der Lage sind, diesen stabil über mehrere Generationen zu exprimieren. Besonders bevorzugt sind dabei rekombinante CHO-Zellen, die vWF oder ein Polypeptid mit vWF-Aktivität, Faktor VIII oder ein Polypeptid mit VIII-Aktivität, vWF und Faktor VIII, Faktor IX oder Faktor II exprimieren.

Der unter serum- und proteinfreien Bedingungen selektionierte erfindungsgemäße Zellklon zeichnet sich insbesondere dadurch, aus, daß er für mindestens 40, vorzugsweise für mindestens 50 Generationen, besonders bevorzugt für mehr als 60 Generationen, in serum- und proteinfreiem Medium in Abwesenheit eines Selektionschuckes stabil ist.

Um eine Masterzellbank anzulegen, werden 30 Generationen benötigt. Um eine durchschnittliche Batchkultur mit 1000'Liter-Maßstab durchzuführen, sind mindestens etwa 40 Generationen erforderlich. Damit ist es erstmals möglich, ausgehend von einem Einzelklon eine "Master cell bank" (MCB), eine "Working cell bank" (WCB) mit etwa 8 bis 10 Generationen und damit eine Zellkultur im Produktionsmaßstab (Produktionsbiomasse) mit bis zu 20 bis 25 Generationen unter diesen Bedingungen herzustellen, da bisherige Zellklone einige Generationen nach Wachstum auf serum- oder proteinfreiem Medium instabil wurden, wodurch a) keine einheitliche Zellkultur mit Produkt-Produzenten und b) keine stabile Produkt-produktivität über eine längeren Zeitraum möglich war.

Der erfindungsgemäße Zellklon ist damit unter Produktionsbedingungen in serum- und proteinfreiem Medium in Abwesenheit eines Selektiondrucks für mindestens 40 Generationen stabil. Bisher beschriebene Verfahren zeigten lediglich eine Generationszahl von weniger als 10 Generationen Produktproduktivität unter proteinfreien Bedingungen (Reiter et al., 1992, supra).

Als Stabilitätskriterium gilt eine Mindestanzahl von mindestens 40 Generationen, vorzugsweise mehr als 50, besonders bevorzugt mehr als 60 Generationen im Produktionsprozeß, während der eine stabile Expression der Proteine stattfindet und sich die Zellen morphologisch-phänotypisch nicht verändern und keine tumorogenen Eigenschaften aufweisen.

Es wurde überraschenderweise gefunden, daß der erfindungsgemäße Zellklon unter serum- und proteinfreien Bedingungen eine erhöhte Produkt-Produktivität selbst im Vergleich zum Ursprungszellklon, der in serumhaltigem Medium kultiviert wurde, aufweist.

Gemäß einem weiteren Aspekt stellt die vorliegende Erfindung eine Zellkultur zur Verfügung, die mindestens 90%, vorzugsweise mehr als 95%, besonders bevorzugt mehr als 98%, stabile rekombinante Zellen enthält, die unter serum- und proteinfreien Bedingungen für mindestens 40 Generationen, insbesondere für mindestens 50 Generationen, stabil sind und rekombinantes Produkt exprimieren.

Unter Zellkultur wird im Rahmen der vorliegenden Erfindung eine Master Cell Bank (MCB), eine Working Cell Bank -(WCB-Arbeitszellbank) oder eine Produktionsbiomasse in einem großtechnischen Produktionsbioreaktor verstanden.

Erfindungsgemäß wird die Zellkultur insbesondere durch Kultivieren eines stabilen rekombinanten Zellklons der oben beschriebenen Art unter serum- und proteinfreien Bedingungen in Abwesenheit eines Selektionsdruckes erhalten.

Die erfindungsgemäße Zellkultur ist dabei erhältlich durch Vermehren des isolierten stabilen Zellklons vom Einzelklon, der Saatzellen bis zur der MCB, der WCB oder einer Biomasse im Produktionsmaßstab im Bioreaktor unter serum- und proteinfreien Bedingungen, bespielsweize ohne Selektionsdruck auf das Selektions- und/oder Markergen. Es hat sich insbesondere gezeigt, daß die rekombinanten Zellen in einer Zellkultur, die ausgehend vom erfindungsgemäßen stabilen rekombinanten Klon erhalten werden, für mindestens 40 Generationen unter serum- und proteinfreien Bedingungen im Abwesenheit eines Selektionsdruckes stabil sind.

Die gemäß der vorliegenden Erfindung zur Verfügung gestellte Zellkultur, die von.einem serum- und proteinunabhängigen stabilen Zellklon hergestellt ist, weist unter proteinfreien Kultivierungs- und Produktionsbedingungen mindestens 90%, vorzugsweise mindestens 95%, besonders bevorzugt mindestens 98%, stabile rekombinante Zellen auf. Unter stabilen rekombinanten Zellen werden dabei insbesondere rekombinante Säugerzellen verstanden, die vom stabilen Zellklon abgeleitet sind. Bevorzugt sind dabei rekombinante CHO-Zellen, vorzugsweise CHO-dhfr⁻-Zellen, CHO-Kl-Zellen, und BHK-Zellen, die einen Blutfaktor, vorzugsweise rekombinanten vWF, Faktor VIII, Faktor VIII und vWF, Faktor IX oder Faktor II exprimieren.

Die erfindungsgemäße Zellkultur kann die stabilen rekombinanten Zellen als Suspensionskultur enthalten. Die Zellen können auch an einen Träger, insbesondere einen Mikroträger, immobilisiert sein, wobei insbesondere poröse Mikroträger bevorzugt sind. Als besonders geeignet haben sich dabei poröse Träger, wie etwa Cytoline® oder Cytopore® , gezeigt.

Gemäß einem weiteren Aspekt stellt die vorliegende Erfindung ein Verfahren zur großtechnischen Herstellung eines rekombinanten Produktes unter serum- und proteinfreien Bedingungen, unter Einsatz des erfindungsgemäßen stabilen Zellklons zur Verfügung. Das Verfahren umfaßt dabei die Schritte des Bereitstellens eines isolierten, stabilen rekombinanten Zellklons der oben beschriebenen Art zur Herstellung einer Zellkultur. Dabei erfolgt die Vermehrung des isolierten.stabilen Zellklons unter serum- und proteinfreien Bedingungen in Abwesenheit eines Selektionsdruckes vom stabilen Einzel-Zellklon bis zur Zellkultur. Insbesondere erfolgt auch die Subkultivierung der stabilen Zellklone unter proteinfreien Bedingungen, insbesondere ohne Zugabe einer Protease, wie etwa Trypsin. Dadurch ist gewährleistet, daß zu keinem Zeitpunkt während der Herstellung einer in der Produktion eines rekombinanten Produktes eingesetzten Zellkultur, eine Kontamination, gegebenfalls verursacht durch die Zugabe von serum- oder proteinhaltigen Zusätzen humanen oder tierischen Ursprungs zur Zellkultur, erfolgt. Damit wird erstmals ein Verfahren beschrieben, das vom Ausgangsklon über die Herstellung einer Working cell bank bis zur Produktionsbiomasse und die anschließende Produktion von rekombinantem Protein unter serum- und proteinfreien Bedingungen in Abwesenheit eines Selektionsdruckes erlaubt.

Die Herstellung der rekombinanten Produkte mit der erfindungsgemäßen Zellkultur, die mehr als 90%, vorzugsweise mehr als 95%, besonders bevorzugt mehr als 98%, stabile Produktproduzenten-Zellen enthält, kann als Suspensionskultur oder mit an Trägerimmobilisierten Zellen erfolgen. Der Prozeß kann dabei im batch-weisen, kontinuierlichen Verfahren oder durch Perfusionstechnik mit serum- und proteinfreiem Medium erfolgen.

Die exprimierten rekombinanten Proteine werden anschließend aus dem Zellkulturüberstand gewonnen, mit aus dem Stand der Technik bekannten Verfahren gereinigt und weiterverarbeitet.

Als serum- und proteinfreies Medium kann jedes bekannte synthetische Medium eingesetzt werden. Konventionelle synthetische Minimalmedien können anorganische Salze, Aminosäuren, Vitamine und eine Kohlehydratquelle und Wasser enthalten. Beipielsweise kann es DMEM/HAM's F12-Medium sein. Der Gehalt an Soja- oder Hefeextrakt kann zwischen 0,1 und 100 g/l, besonders bevorzugt zwischen 1 und 5 g/l liegen. Als besonders bevorzugte Ausführungsform kann Sojaextrakt, beispielsweise Sojapepton verwendet werden. Das Molekulargewicht des Sojapeptons beträgt weniger als 50kD, bevorzugt weniger als 10kD.

Besonders bevorzugt wird ein Medium mit folgender Zusammensetzung verwendet: Synthetisches Minimalmedium (1 bis 25 g/l), Sojapepton (0,5 bis 50 g/l), L-Glutamin (0,05 bis 1 g/l), NaHCO₃ (0,1 bis 10 g/l), Ascorbinsäure (0,0005 bis 0,05 g/l), Ethanolamin (0,0005 bis 0,05 g/l), Na-Selenit (0,0001 bis 0,01 g/l). Dem Medium kann gegebenenfalls ein nichtionisches Oberflächenmittel wie etwa Polypropylenglycol (PLURONIC F-61, PLURONIC F-68, SYNPERONIC F-68, PLURONIC F-71 oder PLURONIC F108j als Entschäumer zugegeben werden.

Dieses Mittel wird allgemein angewendet, um die Zellen vor den negativen Auswirkungen der Belüftung zu schützen, da ohne Zugabe eines Oberflächenmittels die aufsteigenden und zerplatzenden Luftblasen zur Schädigung jener Zellen führen können, die sich an der Oberfläche dieser Luftblasen befinden ("sparging"), (Murhammer und Goochee, 1990, Biotechnol.Prog. 6:142-148)

Die Menge an nichtionischem Oberflächenmittel kann dabei zwischen 0,05 und 10 g/l liegen, besonders bevorzugt ist jedoch eine möglichst geringe Menge zwischen 0,1 und 5 g/l. Weiters kann das Medium auch Cyclodextrin oder ein Derivat davon enthalten.

Der Zusatz von nichtionischem Oberflächenmittel oder von Cyclodextrin ist jedoch nicht erfindungswesentlich. Vorzugsweise enthält das serum- und proteinfreie Medium einen Proteaseinhibitor, wie etwa Serinproteaseinhibitoren, die Gewebekultur-tauglich und synthetischen oder pflanzlichen Ursprungs sind.

Die Parameter für die Kultivierung der Zellen, wie O₂-Konzentration, Perfusionsgeschwindigkeit oder Mediumswechsel, pH, Temperatur und Kultivierungstechnik sind dabei abhängig von den jeweils eingesetzten Zelltypen und können vom Fachmann in einfacher Weise ermittelt werden. Beispielsweise kann die Kultivierung von CHO-Zellen in einem Rührtank und Perfusion mit proteinfreiem Medium mit einer Perfusionsrate von 1 bis 10 Volumenwechsel/Tag, einem pH-Wert zwischen 7,0 und 7,8, vorzugsweise bei 7,4, einer O₂- Konzentration zwischen 40% bis 60%, vorzugsweise bei 50%, und einer Temperatur zwischen 34°C und 38°C, vorzugsweise von 37°C, erfolgen.

Gemäß einem weiteren Aspekt stellt die vorliegende Erfindung ein Verfahren zur Gewinnung eines stabilen rekombinanten Zellklons zur Verfügung, umfassend die Schritte der
- Vermehrung eines rekombinanten Ursprungsklons bis zur Zellkultur in serumhaltigem Medium, ohne Selektionsdruck
- Kultivierung der Zellen unter serum- und proteinfreien, vorzugsweise unter produktionsäquivalenten Bedingungen in Abwesenheit eine Selektionsdrucke
- Testen der Zellkultur unter serum- und proteinfreien Bedingungen auf Produktproduzenten
- Klonieren der stabilen rekombinanten Zellklone unter serum- und proteinfreien Bedingungen, wobei die Klonierung durch allgemein bekannte Techniken, wie Vereinzelung der Zellen durch Ausverdünnen und Anzüchten der Einzelklone erfolgen kann
- Vermehrung der isolierten Zellklone unter serum- und proteinfreien Bedingungen in Abwesenheit eines Selectionsdruckes und
- gegebenenfalls Austesten der Zellkultur auf Produktproduzenten.

Dabei werden nur solche rekombinanten Zellklone als stabil angesehen, die für mindestens 10, vorzugsweise mindestens 20, und insbesondere mindestens 50 Generationen, in proteinfreiem Medium stabil rekombinantes Protein exprimieren.

Die Erfindung wird anhand der nachfolgenden Beispiele beschrieben, wobei sie jedoch nicht auf die Beispiele beschränkt ist.

### Kurze Beschreibung der Figuren

Figur 1: zeigt die mikroskopische Aufnahme einer Arbeitszellbank eines Ursprungsklons zum Zeitpunkt der Umadaptierung von serumhaltigem auf serum- und proteinfreies Medium (A), nach 10 Generationen in serum- und proteinfreiem Medium (B) und nach 60 Generationen in serum- und proteinfreiem Medium (C).
Figur 2: zeigt die mikroskopische Aufnahme einer Zellkultur ausgehend von einem stabilen rekombinanten Zellklon unter serum-und proteinfreien Bedingungen auf der Stufe der Arbeitszellbank (A), nach 10 Generationen (B) und nach 60 Generationen (C)
Figur 3: zeigt die Ergebnisse der Kultivierung eines rFVIII-CHO-Zellklons in einem 10 1-Perfusionsbioreaktor.
   a) FVIII-Aktivität (mEinheiten/ml) und Perfusionsrate (1-5/Tag) über einen Zeitraum von 42 Tagen.
   b) Volumetrische Produktivität (Einheiten Faktor VIII/l/Tag) im Perfusionsbioreaktor.

### Beispiele:

### B e i s p i e l 1: Stabilität von rvWF-CHO-Zellen nach Umstellen von serumhaltigem auf serum- und proteinfreies Medium

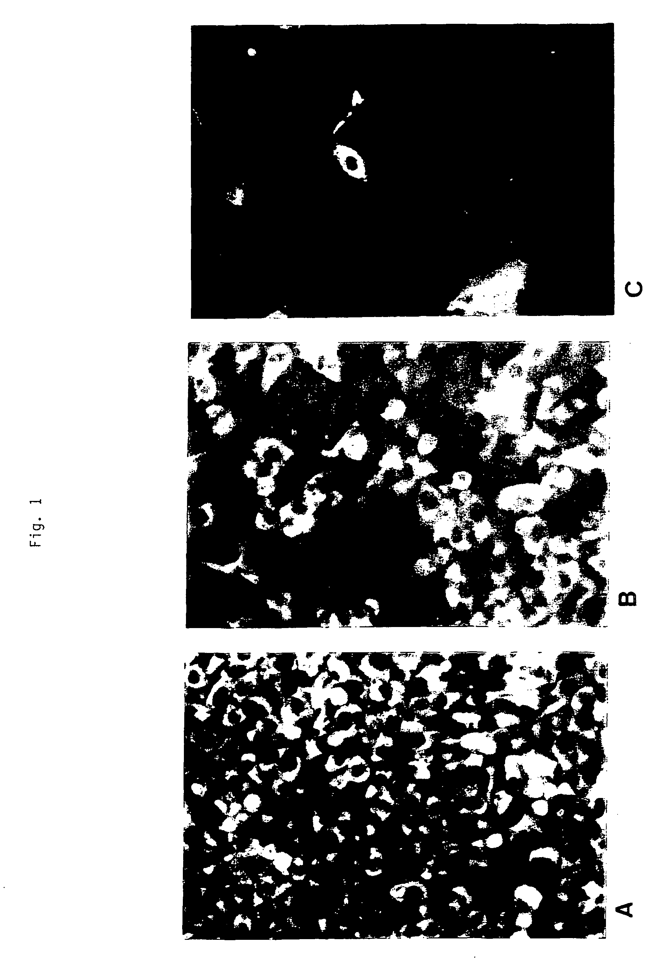

CHO-dhfr⁻-Zellen wurden Plasmid phAct-rvWF und pSV-dhfr, cotransfiziert und vWF-exprimierende Klone, wie in Fischer et al. (1994, FEBS Letters 351:345-348) beschrieben, subkloniert. Von den Subklonen, die stabil rvWF exprimierten, wurde unter serum-haltigen Bedingungen, jedoch in Abwesenheit von MTX, eine Arbeitszellbank (Working cell bank, WCB) angelegt und die Zellen unter serum-haltigen Bedingungen auf einem porösen Mikroträger (Cytopore®) immobilisiert. Nachdem eine Zelldichte von 2x10⁷ Zellen/ml Trägermatrix erreicht wurde, erfolgte die Umstellung der Zellen auf serum- und proteinfreies Medium. Die Zellen wurden für mehrere Generationen unter serum- und proteinfreien Bedingungen weiterkultiviert. Mittels Immunfluoreszenz mit markierten anti-vWF-Antikörpern wurden die Zellen zu verschiedenen Zeitpunkten in serum- und proteinfreiem Medium getestet. Die Bewertung der Stabilität der Zellen erfolgte bei der Arbeitszellbank vor der Mediumsumstellung, nach 10 und 60 Generationen im serum- und proteinfreiem Medium. Während die Arbeitszellbank noch 100% rvWF-Produzenten aufwies (Figur 1 A), sank der Anteil der rvWF-Produzenten nach 10 Generationen in serum- und proteinfreiem Medium auf etwa 50% ab (Figur 1 B). Nach 60 Generationen wurden mehr als 95% der Zellen als Nicht-Produzenten identifiziert (Figur 1 C).

### B e i s p i e l 2 : Klonierung von stabilen rekombinanten CHO-Klonen

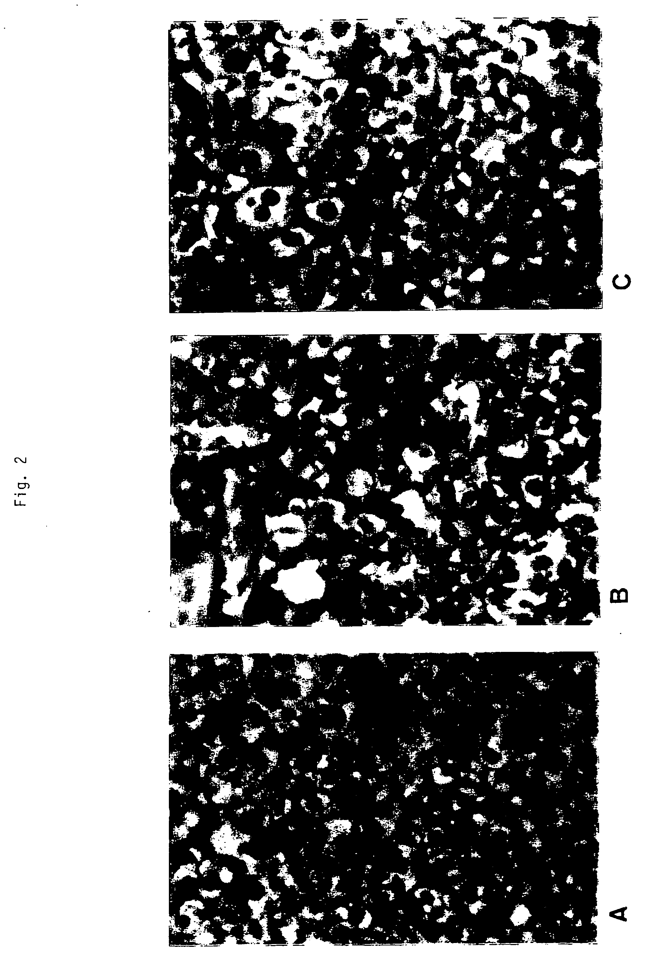

Von der Zellkultur enthaltend rvWF-CHO-Zellen gemäß Beispiel 1, die für 60 Generationen in serum- und proteinfreiem Medium kultiviert worden war (Figur 1 C), wurde eine Verdünnung angelegt und jeweils 0,1 Zellen/well einer Mikrotiterplatte ausgesät. Die Zellen wurden in DMEM/HAM's F12 ohne Serum- oder Proteinzusätze und ohne Selektionsdruck für etwa 3 Wochen kultiviert und die Zellen mit Immunfluoreszenz mit markierten anti-vWF-Antikörpern getestet. Ein als positiv identifizierter Zellklon wurde als Ausgangsklon für die Herstellung einer Saat-Zellbank eingesetzt. Von der Saat-Zellbank wurde eine Master-Zellbank (MCB) in serum-und proteinfreiem Medium angelegt und Einzelampullen für die weitere Herstellung einer Arbeitszellbank weggefroren. Ausgehend von einer Einzelampulle wurde eine Arbeitszellbank in serum- und proteinfreiem Medium hergestellt. Die Zellen wurden auf poröse Mikroträger immobilisiert und für mehrere Generationen unter serum- und proteinfreien Bedingungen weiterkultiviert. Mittels Immunfluoreszenz mit markierten anti-vWF-Antikörpern wurden die Zellen zu verschiedenen Zeitpunkten in serum- und proteinfreiem Medium auf Produktivität getestet. Die Bewertung der Stabilität der Zellen erfolgte auf der Stufe der Arbeitszellbank und nach 10 und 60 Generationen im serum- und proteinfreiem Medium. Sowohl auf Stufe der Arbeitszellbank (Figur 2 A), als auch nach 10 (Figur 2 B) und 60 Generationen (Figur 2 C) wurden annähernd 100% der Zellen als positive stabile rekombinante Klone identifiziert, die rvWF exprimieren.

### B e i s p i e l 3 : Zellspezifische Produktivität der rekombinanten Zellklone

Von der definierten Stufe während der Kultivierung von rekombinanten Zellen wurde eine definierte Zellzahl entnommen und mit frischem Medium für 24 h inkubiert. Die rvWF:Risto-CoF-Aktivität wurde in den Zellkulturüberständen bestimmt. Tabelle 1 zeigt, daß die zellspezifische Produktivität bei den erfindungsgemäßen stabilen rekombinanten Zellklonen auch nach 60 Generationen in serum- und proteinfreiem Medium stabil war, und sogar im Vergleich zum Ursprungsklon, der in serumhaltigem Medium kultiviert war, erhöht war.

**Tabelle 1:**

| Zellklon | zellspezifische Produktivität der Arbeitszellen mU rvWF/10⁶Zellen/Tag | zellspezifische Produktivität nach 10 Generationen mU rvWF/10⁶Zellen/Tag | zellspezifische Produktivität nach 60 Generationen mU rvWF/10⁶Zellen/Tag |
|---|---|---|---|
| rvWF-CHO #808.68 Ursprungszellklon | 55 | 30 | < 10 |
| r-vWF-CHO F7 *) stabiler Klone | 62 | 65 | 60 |

| | | | |
|---|---|---|---|
| *) hinterlegt gemäß Budapester Vertrag am 22.1.1998 (ECACC (European Collection of Cell Cultures, Salisbury, Wiltshire SP4 OJG, UK); Hinterlegungsnummer 98012206) | | | |

### B e i s p i e l 4 : Kultivierung von rFVIII-CHO Zellen in protein- und serumfreiem Minimalmedium

Eine Zellkultur enthaltend rFVIII-CHO-Zellen wurde in einem 10 1-Rührtank und Perfusion kultivert. Dabei wurde ein serum-und proteinfreies Medium verwendet. Die Zellen wurden dabei auf einem porösen Mikroträger (Cytopore®, Pharmacia) immobilisiert und für mindestens 6 Wochen kultiviert. Die Perfusionsrate betrug 4 Volumenwechsel/Tag, der pH-Wert lag bei 6,9-7,2, die O₂₋Konzentration bei etwa 20-50% und die Temperatur bei 37°C.

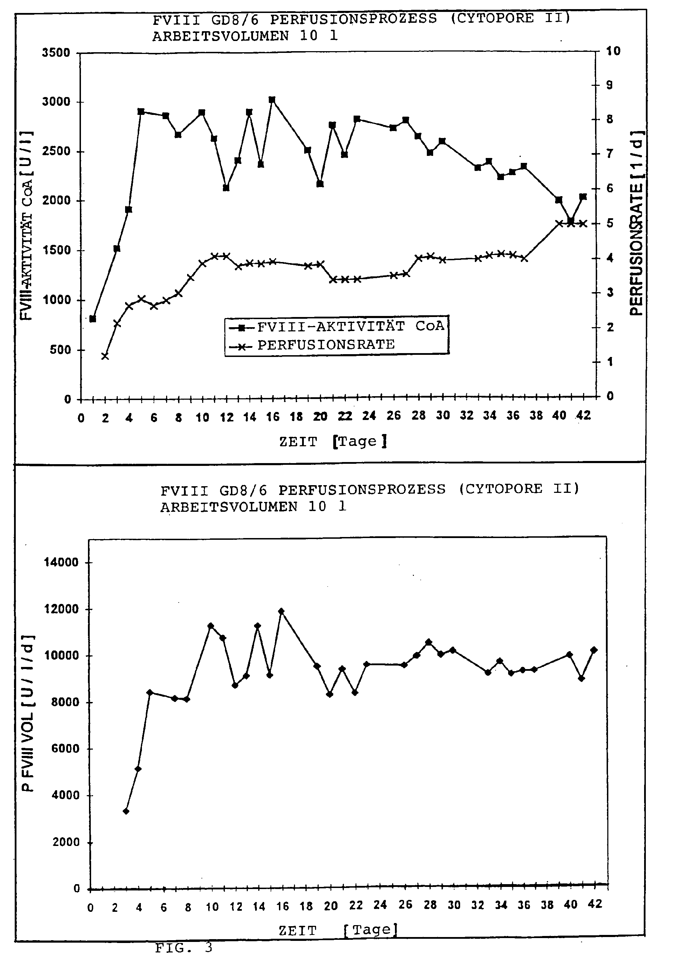

Figur 3 zeigt die Ergebnisse der Kultivierung eines rFVIII-CHO-Zellklons in einem 10 1-Perfusionsbioreaktor.
a) FVIII-Aktivität (mEinheiten/ml) und Perfusionsrate (1-5/Tag) über einen Zeitraum von 42 Tagen.
b) Volumetrische Produktivität (Einheiten Faktor VIII/1/Tag) im Perfusionsbioreaktor.

**Tabelle 2:**

| Kultivierungstage | Zellspezifische Produktivität (mU/10⁶ Zellen/Tag) | Immunfluoreszenz (% FVIII-positive Zellen) |
|---|---|---|
| 15 | 702 | n.a. |
| 21 | 1125 | n.a. |
| 28 | 951 | > 95% |
| 35 | 691 | > 95% |
| 42 | 970 | n.a. |

Tabelle 2 zeigt die Stabilität und spezifische Produktivität der rFVIII-exprimierenden Zellen. Für diese Ergebnisse wurden nach 15, 21, 28, 35 und 42 Tagen Proben entnommen, bei 300 g abzentrifugiert und in frischem serum- und proteinfreien Medium resuspendiert. Nach weiteren 24 Stunden wurde die Faktor VIII-Konzentration in den Zellkulturüberständen und die Zellzahl bestimmt. Ausgehend von diesen Daten wurde die spezifische FVIII-Produktivität berechnet.

Es wurde eine stabile durchschnittliche Produktivität von 888 mEinheiten/10⁶-Zellen/Tag erreicht. Diese stabile Produktivität wurde auch durch Immunfluoreszenz mit markierten anti-FVIII-Antikörpern nach 15, 21, 28, 35 und 42 Tagen in serum- und proteinfreiem Medium bestätigt.

## Patentansprüche

1. Verfahren zur Herstellung eines stabilen rekombinanten Zellklons, der unter Produktionsbedingungen in serum-und proteinfreies Medium für mindestens 40 Generationen stabil ist, **gekennzeichnet durch** die folgenden Schritte:
- Bereitstellen eines rekombinanten Ursprungszellklons,
- Kultivierung des rekombinanten Ursprungszellklons auf serumhaltigem Medium,
- Umadaptieren der Zellen auf serum- und proteinfreies Medium in Abwesenheit eines Selektionsdruckes ,
- Testen der Zellkultur nach Umadaptierung auf in Abwesenheit eines Selektionsdruckes stabile Produkt-Produzenten und
- Klonieren eines stabilen Produkt-Produzenten-Zellklons unter serum-und proteinfreien Bedingungen in Abwesenheit eines Selektionsdruckes .

2. Verfahren nach Anspruch 1, **dadurch gekennzeichnet, daß** nach dem Klonieren der erhaltene stabile Zellklon in isolierter Form vorliegt.

3. Verfahren nach Anspruch 1 oder 2, **dadurch gekennzeichnet, daß** eine rekombinante Säugerzelle als Ursprungsklon bereitgestellt wird.

4. Verfahren nach Anspruch 3, **dadurch gekennzeichnet, daß** die Säugerzelle eine rekombinante CHO-Zelle oder BHK-Zelle ist.

5. Verfahren nach einem der Ansprüche 1 bis 4, **dadurch gekennzeichnet, daß** der rekombinante Zellklon die für ein rekombinantes Polypeptid oder Protein kodierenden Sequenzen enthält.

6. Verfahren nach Anspruch 5, **dadurch gekennzeichnet, daß** das rekombinante Protein ein Blutfaktor, ausgewählt aus der Gruppe bestehend aus Faktor II, Faktor V, Faktor VII, Faktor VIII, Faktor IX, Faktor X, Faktor XI, Protein S, Protein C oder einer aktivierten Form eines der Faktoren, oder vWF ist.

7. Verfahren nach einem der Ansprüche 1 bis 6, **dadurch gekennzeichnet, daß** als Ursprungsklon eine rekombinante CHO-Zelle, die von Willebrand-Faktor exprimiert, bereitgestellt wird.

8. Verfahren nach einem der Ansprüche 1 bis 6, **dadurch gekennzeichnet, daß** als Ursprungsklon eine rekombinante CHO-Zelle, die Faktor VIII exprimiert, bereitgestellt wird.

9. Verfahren nach einem der Ansprüche 1 bis 6, **dadurch gekennzeichnet, daß** als Ursprungsklon eine rekombinante CHO-Zelle, die Faktor VIII und vWF co-exprimiert, bereitgestellt wird.

10. Verfahren nach einem der Ansprüche 1 bis 6, **dadurch gekennzeichnet, daß** als Ursprungsklon eine rekombinante CHO-Zelle, die Faktor IX exprimiert, bereitgestellt wird.

11. Verfahren nach einem der Ansprüche 1 bis 6, **dadurch gekennzeichnet, daß** als Ursprungsklon eine rekombinante CHO-Zelle, die Faktor II exprimiert, bereitgestellt wird.

12. Stabiler rekombinanter Zellklon, erhältlich durch ein Verfahren nach einem der Ansprüch 1 bis 11, **dadurch gekennzeichnet, daß** er unter Produktionsbedingungen in serum-und proteinfreiem Medium in Abwesenheit eines Selektionsdruckes für mendestens 40 Generationen stabil ein rekombinantes Produkt experiment.

13. Zellklon nach Anspruch 12, **dadurch gekennzeichnet, daß** er in isolierter Form vorliegt.

14. Zellklon nach einem der Ansprüche 12 oder 13, **dadurch gekennzeichnet, daß** er von einer rekombinanten Säugerzelle abgeleitet ist.

15. Zellklon nach Anspruch 14, **dadurch gekennzeichnet, daß** die Säugerzelle eine rekombinante CHO-Zelle oder BHK-Zelle ist .

16. Zellklon nach Anspruch 15, **dadurch gekennzeichnet, daß** die Säugerzelle eine rekombinante CHO-Zelle ist.

17. Zellklon nach einem der Ansprüche 12 bis 16, **dadurch gekennzeichnet, daß** er die für ein rekombinantes Polypeptid oder Protein kodierenden Sequenzen enthält.

18. Zellklon nach Anspruch 17, **dadurch gekennzeichnet, daß** das rekombinante Protein ein Blutfaktor, ausgewählt aus der Gruppe bestehend aus Faktor II, Faktor V, Faktor VII, Faktor VIII, Faktor IX, Faktor X, Faktor XI, Protein S, Protein C oder einer aktivierten Form eines der Faktoren, oder vWF ist.

19. Zellklon nach Anspruch 18, **dadurch gekennzeichnet, daß** das rekombinante Protein Faktor VIII ist.

20. Zellklon nach Anspruch 16, **dadurch gekennzeichnet, daß** die Zelle eine rekombinante CHO-Zelle ist, die von Willebrand-Faktor exprimiert.

21. Zellklon nach Anspruch 16, **dadurch gekennzeichnet, daß** die Zelle eine rekombinante CHO-Zelle ist, die Faktor VIII exprimiert.

22. Zellklon nach Anspruch 16, **dadurch gekennzeichnet, daß** die Zelle eine rekombinante CHO-Zelle ist, die Faktor VIII und vWF co-exprimiert.

23. Zellklon nach Anspruch 16, **dadurch gekennzeichnet, daß** die Zelle eine rekombinante CHO-Zelle ist, die Faktor IX exprimiert.

24. Zellklon nach Anspruch 16, **dadurch gekennzeichnet, daß** die Zelle eine rekombinante CHO-Zelle ist, die Faktor II exprimiert.

25. Zellkultur, **dadurch gekennzeichnet, daß** sie durch Kultivieren eines stabilen rekombinanten Zellklons nach Anspruch 12 erhältlich ist.

26. Zellkultur, **dadurch gekennzeichnet, daß** sie mindestens 90% stabile rekombinante Zeleklone nach Anspruch 12 enthält.

27. Zellkultur nach Anspruch 25 oder 26 **dadurch gekennzeichnet, daß** die stabilen rekombinanten Zellklone Säugerzellen sind.

28. Zellkultur nach Anspruch 27, **dadurch gekennzeichnet, daß** die Säugerzellen rekombinante CHO-Zellen sind.

29. Zellkultur nach Anspruch 28, **dadurch gekennzeichnet, daß** die CHO-Zellen CHO-DHFR-Zellen oder CHO-K1-Zellen sind.

30. Zellkultur nach einem der Ansprüche 25 bis 29, **dadurch gekennzeichnet, daß** die stabilen rekombinanten Zellen eine für ein rekombinantes Polypeptid oder Protein kodierende Sequenz enthalten.

31. Zellkultur nach einem der Ansprüche 25 bis 30, **dadurch gekennzeichnet, daß** die stabilen rekombinanten Zellen an einem Mikroträger immobilisiert sind.

32. Verfahren zur großtechnischen Herstellung eines rekombinanten Produktes unter serum- und proteinfreien Bedingungen, **dadurch gekennzeichnet, daß** es die folgenden Schritte umfaßt:
- Bereitstellen eines isolierten, stabilen rekombinanten Zellklons nach Anspruch 12,
- Vermehren des stabilen Zellklons in serum- und proteinfreiem Medium vom Ausgangsklon bis zur Zellkultur in Abwesenheit eines Selektionsdruckes,
- Herstellen der Zellkultur enthaltend stabile Zellen im Bioreaktor in Abwesenheit eines Selektionsdruckes, und
- Ernten der Proteine aus dem Kulturüberstand.

33. Verfahren nach Anspruch 32, **dadurch gekennzeichnet, daß** das serum- und proteinfreie Medium ein synthetisches Minimalmedium enthaltend einen Hefe- oder Sojaextrakt ist.

34. Verfahren nach Anspruch 32 oder 33, **dadurch gekennzeichnet, daß** das Medium Cyclodextrin oder ein Derivat davon enthält.

35. Verfahren nach einem der Ansprüche 32 bis 34, **dadurch gekennzeichnet, daß** das serum- und proteinfreie Medium einen Proteaseinhibitor enthält.

## Claims

1. Method for producing a stable recombinant cell clone, which is stable under production conditions in serum- and protein-free medium for at least 40 generations, **characterised by** the following steps:
- providing a recombinant original cell clone,
- cultivating the recombinant original cell clone on serum-containing medium,
- adapting the cells to serum- and protein-free medium in the absence of a selection pressure,
- testing the cell culture after adaptation for stable product-producers in the absence of a selection pressure and
- cloning a stable product-producer-cell clone in serum- and -protein-free conditions in the absence of a selection pressure.

2. Method according to claim 1, **characterised in that**, after cloning, the stable cell clone obtained is present in isolated form.

3. Method according to claim 1 or 2, **characterised in that** a recombinant mammal cell is provided as the original clone.

4. Method according to claim 3, **characterised in that** the mammal cell is a recombinant CHO cell or BHK cell.

5. Method according to any one of claims 1 to 4, **characterised in that** the recombinant cell clone contains the coding sequences for a recombinant polypeptide or protein.

6. Method according to claim 5, **characterised in that** the recombinant protein is a blood factor, selected from the group consisting of Factor II, Factor V, Factor VII, Factor VIII, Factor IX, Factor X, Factor XI, Protein S, Protein C or an activated form of one of the factors, or vWF.

7. Method according to any one of claims 1 to 6, **characterised in that** a recombinant CHO cell, which expresses von Willebrand factor, is provided as the original clone.

8. Method according to any one of claims 1 to 6, **characterised in that** a recombinant CHO cell, which expresses Factor VIII, is provided as the original clone.

9. Method according to any one of claims 1 to 6, **characterised in that** a recombinant CHO cell, which co-expresses Factor VIII and vWF, is provided as the original clone.

10. Method according to any one of claims 1 to 6, **characterised in that** a recombinant CHO cell, which expresses Factor IX, is provided as the original clone.

11. Method according to any one of claims 1 to 6, **characterised in that** a recombinant CHO cell, which expresses Factor II, is provided as the original clone.

12. Stable recombinant cell clone, obtainable by a method according to any one of claims 1 to 11, **characterised in that** under production conditions in serum- and protein-free medium, in the absence of a selection pressure, stable for at least 40 generations, it expresses a recombinant product.

13. Cell clone according to claim 12, **characterised in that** it is present in isolated form.

14. Cell clone according to either of claims 12 or 13, **characterised in that** it is derived from a recombinant mammal cell.

15. Cell clone according to claim 14, **characterised in that** the mammal cell is a recombinant CHO cell or BHK cell.

16. Cell clone according to claim 15, **characterised in that** the mammal cell is a recombinant CHO cell.

17. Cell clone according to any one of claims 12 to 16, **characterised in that** it contains the coding sequences for a recombinant polypeptide or protein.

18. Cell clone according to claim 17, **characterised in that** the recombinant protein is a blood factor, selected from the group consisting of Factor 11, Factor V, Factor VII, Factor VIII, Factor IX, Factor X, Factor XI, Protein S, Protein C or an activated form of one of the factors, or vWF.

19. Cell clone according to claim 18, **characterised in that** the recombinant protein is Factor VIII.

20. Cell clone according to claim 16, **characterised in that** the cell is a recombinant CHO cell, which expresses von Willebrand factor.

21. Cell clone according to claim 16, **characterised in that** the cell is a recombinant CHO cell, which expresses Factor VIII.

22. Cell clone according to claim 16, **characterised in that** the cell is a recombinant CHO cell, which co.-expresses Factor VIII and vWF.

23. Cell clone according to claim 16, **characterised in that** the cell is a recombinant CHO cell, which expresses Factor IX.

24. Cell clone according to claim 16, **characterised in that** the cell is a recombinant CHO cell, which expresses Factor II.

25. Cell culture, **characterised in that** it can be obtained by cultivating a stable recombinant cell clone according to claim 12.

26. Cell culture, **characterised in that** it contains at least 90% stable recombinant cell clones according to claim 12.

27. Cell culture according to claim 25 or 26, **characterised in that** the stable recombinant cell clones are mammal cells.

28. Cell culture according to claim 27, **characterised in that** the mammal cells are recombinant CHO cells.

29. Cell culture according to claim 28, **characterised in that** the CHO cells are CHO-DHFR cells or CHO-K1 cells.

30. Cell culture according to any one of claims 25 to 29, **characterised in that** the stable recombinant cells contain a coding sequence for a recombinant polypeptide or protein.

31. Cell culture according to any one of claims 25. to 30, **characterised in that** the stable recombinant cells are immobilised on a microsupport.

32. Method for industrial production of a recombinant product under serum- and protein-free conditions, **characterised in that** it comprises the following steps:
- providing an isolated, stable recombinant cell clone according to claim 12,
- propagating the stable cell clone in serum- and protein-free medium from the initial clone up to the cell culture in the absence of a selection pressure,
- producing the cell culture containing stable cells in the bioreactor in the absence of a selection pressure, and
- harvesting the proteins from the culture supernatant.

33. Method according to claim 32, **characterised in that** the serum- and protein-free medium is a synthetic minimal medium containing a yeast or soya extract.

34. Method according to claim 32 or 33, **characterised in that** the medium contains cyclodextrin or a derivative thereof.

35. Method according to any one of claims 32 to 34, **characterised in that** the serum- and protein-free medium contains a protease inhibitor.

## Revendications

1. Procédé de fabrication d'un clone cellulaire recombinant stable, qui est stable pendant au moins 40 générations dans des conditions de production dans un milieu sans sérum ni protéine, **caractérisé par** les étapes suivantes :
- préparation d'un clone cellulaire originel recombinant,
- culture dudit clone cellulaire originel recombinant dans un milieu contenant du sérum,
- adaptation des cellules à un milieu sans sérum ni protéine, en l'absence de pression sélective,
- test de la culture cellulaire après adaptation, en l'absence de pression sélective, à la recherche de productrices de produit stable et
- clonage d'un clone cellulaire producteur de produit stable dans des conditions sans sérum ni protéine, en l'absence de pression sélective.

2. Procédé selon la revendication 1, **caractérisé en ce que** le clone cellulaire stable obtenu après le clonage est présent sous forme isolée.

3. Procédé selon la revendication 1 ou 2, **caractérisé en ce qu'**une cellule de mammifère recombinante est fournie à titre de clone originel.

4. Procédé selon la revendication 3, **caractérisé en ce que** ladite cellule de mammifère est une cellule CHO ou une cellule BHK recombinée.

5. Procédé selon l'une des revendications 1 à 4, **caractérisé en ce que** le clone cellulaire recombiné contient les séquences codant pour un polypeptide ou une protéine recombinant(e).

6. Procédé selon la revendication 5, **caractérisé en ce que** la protéine recombinante est un facteur sanguin, choisi dans le groupe constitué par le facteur II, le facteur V, le facteur VII, le facteur VIII, le facteur IX, du facteur X, le facteur XI, la protéine S, la protéine C, ou une forme activée d'un de ces facteurs, ou le vWF.

7. Procédé selon l'une des revendications 1 à 6, **caractérisé en ce qu'**on fournit à titre de clone originel une cellule CHO recombinante qui exprime le facteur de von Willebrand.

8. Procédé selon l'une des revendications 1 à 6, **caractérisé en ce qu'**on fournit à titre de clone originel une cellule CHO recombinante qui exprime le facteur VIII.

9. Procédé selon l'une des revendications 1 à 6, **caractérisé en ce qu'**on fournit à titre de clone originel une cellule CHO recombinante qui co-exprime le facteur VIII et le vWF.

10. Procédé selon l'une des revendications 1 à 6, **caractérisé en ce qu'**on fournit à titre de clone originel une cellule CHO recombinante qui exprime le facteur IX.

11. Procédé selon l'une des revendications 1 à 6, **caractérisé en ce qu'**on fournit à titre de clone originel une cellule CHO recombinante qui exprime le facteur II.

12. Clone cellulaire recombiné stable, susceptible d'être obtenue selon le procédé de l'une des revendications 1 à 11, **caractérisé en ce qu'**il exprime de façon stable, pendant au moins 40 générations, un produit recombiné dans des conditions de production sans sérum ni protéine, en l'absence de pression sélective.

13. Clone cellulaire selon la revendication 12, **caractérisé en ce qu'**il est présent sous forme isolée.

14. Clone cellulaire selon la revendication 12 ou 13, **caractérisé en ce qu'**il est dérivé d'une cellule de mammifère recombinante.

15. Clone cellulaire selon la revendication 14, **caractérisé en ce que** la cellule de mammifère est une cellule CHO ou une cellule BHK recombinante.

16. Clone cellulaire selon la revendication 15, **caractérisé en ce que** la cellule de mammifère est une cellule CHO recombinante.

17. Clone cellulaire selon l'une des revendications 12 à 16, **caractérisé en ce qu'**il contient les séquences codant pour un polypeptide ou une protéine recombinant(e).

18. Clone cellulaire selon la revendication 17, **caractérisé en ce que** la protéine recombinante est un facteur sanguin choisi dans le groupe constitué par le facteur II, le facteur V, le facteur VII, le facteur VIII, le facteur XIII, le facteur IX, le facteur X, le facteur XI, la protéine S, la protéine C ou une forme activée de l'un des facteurs, ou le vWF.

19. Clone cellulaire selon la revendication 18, **caractérisé en ce que** la protéine recombinante est le facteur VIII.

20. Clone cellulaire selon la revendication 16, **caractérisé en ce que** la cellule est une cellule CHO recombinante qui exprime le facteur von Willebrand.

21. Clone cellulaire selon la revendication 16, **caractérisé en ce que** la cellule est une cellule CHO recombinante qui exprime le facteur VIII.

22. Clone cellulaire selon la revendication 16, **caractérisé en ce que** la cellule est une cellule CHO recombinante, qui co-exprime les facteurs VIII et vWF.

23. Clone cellulaire selon la revendication 16, **caractérisé en ce que** la cellule est une cellule CHO recombinante qui exprime le facteur IX.

24. Clone cellulaire selon la revendication 16, **caractérisé en ce que** la cellule est une cellule CHO recombinante qui exprime le facteur II.

25. Culture cellulaire susceptible d'être obtenue par culture d'un clone cellulaire recombinant stable selon la revendication 12.

26. Culture cellulaire **caractérisée en ce qu'**elle contient au moins 90% de clone cellulaire recombinant stable selon la revendication 12.

27. Culture cellulaire selon la revendication 25 ou 26, **caractérisée en ce que** les clones cellulaires recombinants stables sont des cellules de mammifère.

28. Culture cellulaire selon la revendication 27, **caractérisée en ce que** les cellules de mammifère sont des cellules CHO recombinantes.

29. Culture cellulaire selon la revendication 28, **caractérisée en ce que** les cellules CHO sont des cellules CHO-DHFR ou des cellules CHO-K1.

30. Culture cellulaire selon l'une des revendications 25 à 29, **caractérisée en ce que** les cellules recombinantes stables contiennent une séquence codant pour un polypeptide ou une protéine recombiné(e).

31. Culture cellulaire selon l'une des revendications 25 à 30, **caractérisée en ce que** les cellules recombinantes stables sont immobilisées sur un microsupport.

32. Procédé pour la production technique d'un produit recombinant dans des conditions sans sérum ni protéine, **caractérisé en ce qu'**il comprend les étapes suivantes :
- Fourniture d'un clone cellulaire recombinant stable et isolé selon la revendication 12,
- multiplication du clone cellulaire stable dans un milieu sans sérum ni protéine depuis le clone de départ jusqu'à la culture cellulaire, en l'absence de pression sélective,
- fabrication d'une culture cellulaire contenant des cellules stables dans le bioréacteur, en l'absence d'une pression sélective, et
- collecte des protéines à partir du surnageant de culture.

33. Procédé selon la revendication 32, **caractérisé en ce que** ledit milieu sans sérum ni protéine est un milieu minimal synthétique contenant un extrait de levure ou de soja.

34. Procédé selon l'une des revendications 32 à 33, **caractérisé en ce que** le milieu contient de la cyclodextrine ou un dérivé de celle-ci.

35. Procédé selon l'une des revendications 32 à 34, **caractérisé en ce que** le milieu sans sérum ni protéine contient un inhibiteur de protéase.
